# EUROPEAN PATENT APPLICATION

(11) **EP 3 598 968 A1**
(43) Date of publication of application: **29.01.2020**
(21) Application number: 18461589.6
(22) Date of filing: 23.07.2018
(51) Int. Cl.: A61K 9/16, A61K 9/20, A61K 31/439

(54) **SOLID PHARMACEUTICAL CYTISINE COMPOSITION**

(71) Applicant: Adamed Pharma S.A., 05-152 Czosnow (PL)
(72) Inventor: GARBERA, Kamil, 59-220 Legnica (PL); RZASA, Joanna, 35-116 Rzeszow (PL)
(74) Representative: Sulikowski, Daniel

(57) **Abstract**

The present invention relates to a solid pharmaceutical composition of cytisine for oral administration in the form of a granulate comprising cytisine, a binder and a porous carrier and a method for the preparation of the granulate.

## Description

### Field of the invention

The present invention relates to a solid pharmaceutical composition of cytisine for oral administration in the form of a granulate and a method for the preparation of the granulate.

### Prior art

Cytisine is a known plant alkaloid that is used among others as an anti-smoking medicament for oral administration. Dosage strength of cytisine tablets and capsules for oral administration is 1.5 mg per single unit dose.

Prior art discloses solid pharmaceutical cytisine compositions in the form of a simple blend that can be used for filling into capsules or preparation of tablets by direct compression, without granulation.

EP1586320 describes cytisine solid compositions in the form of a blend that can be used for the preparation of tablets by direct compression. To achieve high homogeneity of cytisine distribution with high stability and good disintegration, tablets comprise 0.5 to 9.0 percent of cytisine, lactose and microcrystalline cellulose at a ratio of 1:2.0 to1:2.5, total content of lactose and microcrystalline cellulose ranging from 87.0 to 97.0 percent by weight of the tablet mass. Average dimension of cytisine particles is 90 µm, dimensions of lactose monohydrate particles is up to 500 µm, and average dimension of microcrystalline cellulose particles is 90 µm.

WO2014076680 discloses cytisine hard capsule form, comprising a blend of micronized cytisine with 100% of particles having a diameter below 10 µm, and microcrystalline cellulose with 99% of particles having a diameter below 38 µm, prepared by simple blending of the components. The mixture is free of lactose that is described as deleterious for chemical stability of cytisine and deleterious for patients with lactose intolerance.

EP2957280 discloses solid cytisine pharmaceutical composition in the form of tablets prepared by direct compression of a tableting mass, comprising 20 to 75% by weight of microcrystalline cellulose as a carrier, micronized cytisine, wherein at least 60% of cytisine particles have size from 10 µm to 200 µm, a diluent selected from mannitol, colloidal silicon dioxide and calcium hydrogen phosphate, a disintegrant, a binder, anti-adhesion agent and a glidant.

### Summary of the invention

Relatively low cytisine content in a unit dosage form causes problems with proper homogeneity of and uniform distribution of the active substance in the composition used for tableting or filling the capsules. When a small amount of an active substance is to be mixed with a large amount of a diluent/carrier, geometric dilution should be used to ensure the uniform distribution of the active substance in the blend. Still, as found by the present inventor, there is a problem with maintaining uniformity of the blend in time because of the segregation in time of the blend, especially when particles sizes of the active substance and the carrier differ.

Another problem is the influence of the size of the active substance on the dissolution and hence bioavailability. Known solid compositions rely on using micronized cytisine to achieve proper homogeneity of tableting mass and even distribution of cytisine, and proper cytisine dissolution. This involves the necessity of careful control of the particle size of cytisine or both cytisine and microcrystalline cellulose carrier. Micronization of cytisine is necessary, this making the process of preparation more complex and costly.

The invention solves the problem of obtaining a stable, uniform and well-soluble cytisine solid composition problem by providing a solid pharmaceutical composition of cytisine in the form of a granulate comprising cytisine, a binder and a carrier that is obtained by wet-granulation. The invention provides also a process form of process for the preparation of solid pharmaceutical cytisine in the form of a granulate, the process comprising wet granulation with a solution of cytisine and a binder as a granulating liquid.

### Detailed description of the invention

According to the invention, there is provided a solid pharmaceutical composition in the form of a granulate comprising cytisine, a binder and a porous carrier (intragranular components). The granulate of the invention is obtained by wet-granulation.

According to the invention, there is also provided a blend comprising the granulate of the invention and further extragranular pharmaceutical excipients.

According to the invention, there is also provided a single unit dosage form selected from the group consisting of a tablet, coated tablet, capsule and sachet, comprising the granulate of the invention and optionally further extragranular pharmaceutical excipients.

The amount of cytisine in the granulate of the invention is in the range from 0.5 to 10% by weight with respect to the weight of the granulate.

The amount of the porous carrier in the granulate of the invention is in the range from 10 to 92% by weight with respect to the total weight of the granulate.

The balance of the weight of the granulate to total 100% is the binder.

It will be appreciated by a skilled person that the carrier in the granulate of the invention is free of lactose and microcrystalline cellulose.

The carrier is preferably a porous carrier having high adsorbent capacity.

The porous carrier preferably has the following characteristics:
- mean particle size distribution (PSD) in the range from 50 to 150 µm;
- specific surface area in the range from 10 to 450 m²/g;
- bulk density in the range from 0.15 to 0.8 g/cm³;

Mean particle size distribution (PSD) can be determined using analytical sieving, Sieve test, Ph. Eur. Method 2.9.12.

Specific surface area can be determined using the test of specific surface area by gas adsorption, Ph.Eur. 9.0, Method 2.9.26.

Bulk density can be determined using the test of Bulk density and tapped density of powders, Ph.Eur. 9.0, Method 2.9.34, or the test of Pycnometric density of solids, Ph.Eur. 9.0, Method 2.9.23.

The porous carrier can be an inorganic one, such as for example magnesium aluminum silicate, silica, such as colloidal silica, such as mesoporous colloidal silica, titanium dioxide, such as nanoporous titanium dioxide. A preferred inorganic porous carrier is magnesium aluminum silicate.

The porous carrier can be also an organic one, such as for example ethylene vinyl acetate copolymer (macroporous) or polypropylene foam powder (microporous).

Besides the porous carrier, the granulate of the invention can comprise further carrier, such as for example mannitol, maize (corn) starch, pregelatinized starch, calcium hydrogen phosphate and their mixtures.

The binder for the granulate can be selected from hydroxypropylmethylcellulose (hypromellose), methylcellulose, polyvinyl alcohol, hydroxyethylcellulose, macrogole. A preferred binder for the granulate is hydroxypropylmethylcellulose (hypromellose). Another preferred binder for the granulate is polyvinyl alcohol. Polyvinylpyrrolidone and arabic gum cannot be used according to negative impact on Cytisine stability.

The excipients for extragranular phase comprise carriers, binders, disintegrants, lubricants, and glidants and their mixtures. Carrier for extragranular phase may be selected from mannitol, sorbitol, xylitol, maltitol, erythritol, isomalt, lactitol, calcium phospate, dibasic calcium phosphate, tribasic calcium phosphate, starch, modified starch, magnesium carbonate, magnesium oxide, calcium carbonate. Binders for extragranular phase may be selected from hypromellose, methylcellulose, polyvinyl alcohol, hydroxyethylcellulose, macrogole. Disintegrants for extragranular phase may be selected from carboxymethyl cellulose, sodium starch glycolate, crospovidone, L- hydroxypropylcellulose (low substituted). Lubricants for extragranular phase may be selected from magnesium stearate, stearic acid, calcium stearate, zinc stearate, glyceryl behenate. Glidants for extragranular phase may be selected from talk, silica collloidal anhydrous and starch.

According to the invention, there is also provided a process for the preparation of cytisine solid pharmaceutical composition comprising a granulate as defined above, said process comprising
a) preparing a granulating liquid by dissolution of cytisine and a binder in a solvent to prepare the granulating liquid;
b) granulating a carrier with the granulating liquid to prepare the granulate.

The step b) of preparing the granulate can include conventional processing steps known in the art of wet granulation, that is drying and sieving of the granulate.

Optionally, in a further step c) extragranular excipients are added to and mixed with the granulate to obtain a blend.

In one embodiment of the process of the invention, the solvent is water. Cytisine is very well soluble in water (43.9 g / 100 g of water at 16°C).

In another embodiment of the process of the invention, the solvent is an organic solvent wherein cytisine easily dissolves and having low boiling point, preferably below 100°C, such as acetone (solubility 7.7 g /100 g at 20°C), ethyl acetate (10 g/ 100 g at 20°C), or ethanol (28.6 g/100 g at 20°C). Preferred organic solvent is ethanol.

A skilled person will be able to easy determine the amount of the solvent needed for dissolution of cytisine to prepare the granulating liquid.

Techniques and apparatuses for performing wet-granulation are known in the art.

In the wet granulation process, agglomeration of the active ingredient and excipients occurs to obtain a mass of larger granulate particles. The granulating liquid comprising cytisine and the binder is fed, especially sprayed, onto the carrier particles as they are agitated in a closed container with blending tools and a chopper, usually in a high-shear mixer or fluidized bed. Dense granules are formed through the liquid and solid bridges that result in the granulate having compact structure and high bulk density. The granulating liquid can be simply poured into the carrier. For improved dose uniformity and obtain a more even granulate, the granulating liquid can be fed by spraying onto the carrier using a spray nozzle.

The granulation can be performed by high-shear granulation. In high-shear granulation, wherein dry powder of the carrier is placed in a mixing bowl usually containing an impeller, which revolves on a horizontal plane, and a chopper, which rotates either in the vertical or horizontal plane. The dry powders are mixed by the rotating impeller before the granulating liquid binder is sprayed onto the top of the bed of powder. Agitation is maintained by the rotating impeller until a predetermined optimum endpoint is reached. The granules are then usually sieved, transferred to another piece of equipment, such as for example a fluidized-bed dryer, for drying, and finally sieved.

The granulation can be also performed by fluidized bed wet granulation, wherein granulating liquid is fed by spraying onto the carrier particles powder bed that is maintained in a fluidized state such as by a flow of air injected at the base of the granulator. The advantage of the fluidized bed wet granulation is that drying can be performed within the same apparatus.

Thus obtained granulate or the blend of the granulate with extragranular excipients can be further processed into a unit dosage tablet form by compression, or coated tablet by compression and coating. Thus obtained granulate of the invention or the blend of the granulate with extragranular excipients can be also filled into a capsule or a sachet unit dosage form.

In one embodiment of the process of the invention the solvent used to prepare the granulating solution is water.

In another embodiment of the process of the invention the solvent used to prepare the granulating liquid is an organic solvent.

### Examples

Magnesium aluminium silicate used in the Examples described below has the following characteristics:
particle size: 60 - 120 µm
Specific surface area: 300 m²/g
bulk density: 0.15 g/cm³

### Example 1. Preparation of the composition of the invention

Granulate of the invention and coated tablet of the invention were prepared. The composition of the granulate and the coated tablet is presented in Table 1 below.

**Table 1.**

| | Ingredient | Amount | | Amount | |
|---|---|---|---|---|---|
| | | [mg/tab] | [%/tab] | [mg/gran] | [%/gran] |
| intragranular | Cytisine | 1.50 | 2.50 | 1.50 | 3.15 |
| | Hydroxypropylmethylcellulose | 1.00 | 1.67 | 1.00 | 2.11 |
| | Mannitol | 30.00 | 50.00 | 30.00 | 63.20 |
| | Corn starch | 10.00 | 16.67 | 10.00 | 21.05 |

| | Ingredient | Amount | | Amount | |
|---|---|---|---|---|---|
| | | [mg/tab] | [%/tab] | [mg/gran] | [%/gran] |
| | Magnesium Aluminium Silicate | 5.00 | 8.33 | 5.00 | 10.52 |
| | Water, purified* | q.s. | q.s. | q.s. | q.s. |
| extragranular | Colloidal silica, anhydrous | 0.60 | 1.00 | | |
| | Hydroxypropylmethylcellulose | 1.20 | 2.00 | | |
| | Mannitol | 9.50 | 15.83 | | |
| | Magnesium stearate | 1.20 | 2.00 | | |
| | Total core | 60.00 | 100.00 | | |
| coating | Film coating system** | 3.00 | | | |
| | Total mass of coated tablet | 63.00 | | | |

| | | | | | |
|---|---|---|---|---|---|
| *Solvent is removed during the drying process and is not present in the finish product. **Coating has been applied for aesthetic reasons. Exemplary film coating can contain AquaPolish P green Project 16712, Menthol flavor powder (SC222873) and aspartame. | | | | | |

Granulates were prepared by high-shear granulation using cytisine aqueous solution as a granulating liquid.

The manufacturing process of cytisine granulate consisted of the following steps:
1. Weighing and sieving of granulate components: weigh specified amounts of intragranular components mannitol, corn starch and magnesium aluminium silicate into separate containers.
2. Preparation of granulation liquid: add a specified amount of a binder into a container with purified water while stirring, and stir until clear solution is obtained. Then add a specified amount of cytisine into the container while stirring, and stir until clear, transparent solution is obtained.
3. High-shear mixing: load and mix granulate components.
4. Granulation liquid addition: add granulation liquid into granulate components blend.
5. Wet massing: mix wet granulate with high speed for additional period of time.
6. Wet granulate sieving: pass wet granulate through a screen.
7. Fluid bed drying: load wet granules into fluid bed drier to remove water added in the granulation stage.
8. Granulate screening: pass dried granulate through a screen to calibrate the granulate.

Granulate was then tableted according to the following procedure.
9. Weighing of extragranular components: weigh specified amounts of extragranular excipients.
10. Sieving I: pass weighted amounts of additional components (except lubricant) through a screen into the container with a granulate.
11. Blending: blend all components to obtain uniform blend without a lubricant.
12. Sieving II: pass weighted amount of lubricant through a screen into the container.
13. Final Blending: blend all components to obtain final blend.
14. Tableting: adjust the machine and run the compression process to obtain tablet cores of the required characteristics having cytisine strength of 1.5 mg.
15. Weighing of coating components: weigh specified amounts of film-coating suspension ingredients.
16. Film-coating suspension preparation: water purified is stirred to form a vortex. Then additional ingredients are added to the stirring container. All ingredients are stirred together until a homogeneous suspension is obtained.
17. Film-coating: spray initially preheated and dedusted tablet cores with film-coating suspension until desired weight gain is obtained.

Average contents and uniformity for three pilot series of thus obtained tablets having the cytisine strength of 1.5 mg were determined at the beginning, in the middle at the end of the tabletting process. The results of the average contents and Acceptance Values (AV) calculated according to European Pharmacopoeia are presented in Table 1 below.

**Table 1. Cytisine average content and uniformity of content for Cytisine tablets for exemplary batches.**

| **Batch** | **Beginning** | | **Middle** | | **End** | |
|---|---|---|---|---|---|---|
| | **Average content [%]** | **AV** | **Average content [%]** | **AV** | **Average content [%]** | **AV** |
| 1 | 101.5 | 2.6 | 101.2 | 2.4 | 101.1 | 4.1 |
| 2 | 100.9 | 4.8 | 100.5 | 1.7 | 100.3 | 1.7 |
| 3 | 102.5 | 4.8 | 101.5 | 2.6 | 101.9 | 2.1 |

Acceptance values are within prescribed limits, even at the end of the tabletting process.

### Example 2. Test of chemical stability

Chemical stability during storage of the tablets according to Example 1 was determined in comparison with prior art compositions. The results are presented in Table 2.

As can be seen, chemical stability profile of the compositions of the invention is significantly better than for two prior art products irrespective on storage conditions. Quality of the product of the invention is adequate after 6 months storage in accelerated conditions (40°C, 75%RH) in contrast to presented reference products.

**Table 2. Comparison of stability studies for composition of the invention and prior art compositions**

| Batch | | Example 1 | | | | EP1586320, Ex. 2 | | | | WO2014/076680, Ex. 1 | | | |
|---|---|---|---|---|---|---|---|---|---|---|---|---|---|
| **Storage conditions** | | 40°C / 75%RH | | | | | | | | | | | |
| **Time point** | | 0 | 1.5M | 3M | 6M | 0 | 1.5M | 3M | 6M | 0 | 1.5M 3M | | 6M |
| **Imp.** | Max Unk. imp. | <LOQ | 0.05 | 0.03 | 0.05 | 0.45 | | 12.68 | 17.69 | <0.05 | | 0.10 | 0.19 |
| | Impurity FO | <LOQ | 0.06 | 0.11 | 0.26 | 0.05 | | 6.44 | 9.30 | <0.05 | | 0.26 | 1.07 |
| | Impurity ME | <LOQ | <LOQ | 0.03 | 0.05 | <LOQ | | 0.42 | 0.42 | <LOQ | | 0.07 | 0.14 |
| | **Total imp.** | **<LOQ** | **0.12** | **0.17** | **0.40** | **0.50** | | **21.59** | **31.12** | **<0.05** | | **0.43** | **1.59** |

| **Storage conditions** | | 30°C / 65%RH | | | | | | | | | | | |
|---|---|---|---|---|---|---|---|---|---|---|---|---|---|
| **Time point** | | 0 | 1.5M | 3M | 6M | 0 | 1.5M | 3M | 6M | 0 | 1.5 M | 3M | 6M |
| I**mp.** | Max Unk. imp. | <LOQ | 0.04 | 0.04 | 0.05 | 0.45 | | 2.32 | 3.67 | <0.05 | | | 0.09 |
| | Impurity FO | <LOQ | <LOQ | <LOQ | 0.05 | 0.05 | | 1.06 | 2.32 | <0.05 | | | 0.08 |
| | Impurity ME | <LOQ | <LOQ | <LOQ | <LOQ | <LOQ | | 0.15 | 0.32 | <LOQ | | | <LOQ |
| | **Total imp.** | **<LOQ** | **0.04** | **0.04** | **0.10** | **0.50** | | **3.71** | **7.00** | **<0.05** | | | **0.27** |

| **Storage conditions** | | 25°C / 60%RH | | | | | | | | | | | |
|---|---|---|---|---|---|---|---|---|---|---|---|---|---|
| **Time point** | | 0 | 1.5M | 3M | 6M | 0 | 1.5M | 3M | 6M | 0 | 1.5 M | 3M | 6M |
| **Imp.** | Max Unk. imp. | <LOQ | 0.04 | 0.04 | 0.05 | 0.45 | | 1.40 | 1.80 | <0.05 | | | 0.09 |
| | Impurity FO | <LOQ | <LOQ | <LOQ | <LOQ | 0.05 | | 0.38 | 0.84 | <0.05 | | | 0.04 |
| | Impurity ME | <LOQ | <LOQ | <LOQ | <LOQ | <LOQ | | 0.06 | 0.14 | <LOQ | | | <LOQ |
| | **Total imp.** | **<LOQ** | **0.04** | **0.04** | **0.05** | **0.50** | | **1.88** | **2.90** | **<0.05** | | | **0.20** |

| | | | | | | | | | | | | | |
|---|---|---|---|---|---|---|---|---|---|---|---|---|---|
| LOQ - limit of Quantification; M - months; Imp. - impurity; Max Unk. Imp - maximum unknown impurity | | | | | | | | | | | | | |

Example 3. Further compositions of the invention were prepared according to the procedure described in Example 1.

### Example 3A. Granulate and tablet

Granulate was prepared in a manner described in Example 1 from the following intragranular ingredients specified in Table 3A. Then to the granulate the following extragranular ingredients were added and blended. The blend was tableted to obtain 60 mg tablets having having cytisine strength of 1.5 mg or 120 mg having cytisine strength of 3 mg.

**Table 3A**

| | Ingredient | Functionality | Amount | |
|---|---|---|---|---|
| | | | [mg/granulate] | [%/granulate] |
| Intragranular phase | Cytisine | Active substance | 1.5 | 3.75 |
| | Hypromellose | Binder | 2.0 | 5.00 |
| | Magnesium Aluminium Silicate | Carrier / Diluent | 36.5 | 91.25 |
| | Water, purified | Solvent* | q.s. | q.s. |
| | Total mass of granulate | | 40.00 | 100.00 |
| Extragranular phase | Silica colloidal, anhydrous | glidant | 0.6 | |
| | Mannitol | diluent | 18.2 | |
| | Magnesium stearate | lubricant | 1.2 | |

| | | | | |
|---|---|---|---|---|
| *Solvent is removed during the drying process and is not present in the finish product. | | | | |

### Example 3B. Granulate

Granulate was prepared in a manner described in Example 1 from the following intragranular ingredients.

| | **Ingredient** | **Functionality** | **Amount** | |
|---|---|---|---|---|
| | | | **[mg/granulate]** | **[%/granulate]** |
| Intragranular phase | Cytisine | Active substance | 1.5 | 3.00 |
| | Hypromellose | Binder | 0.5 | 1.00 |
| | Mannitol | Carrier / Diluent | 28.0 | 56.00 |
| | Magnesium Aluminium Silicate | Carrier / Diluent | 20.0 | 40.00 |
| | Ethanol | Solvent* | - | - |
| | **Total** | | **50.00** | **100.00** |

| | | | | |
|---|---|---|---|---|
| * Solvent is removed during the drying process and is not present in the finish product. | | | | |

### Example 3C.

Granulate was prepared in a manner described in Example 1 from the following intragranular ingredients.

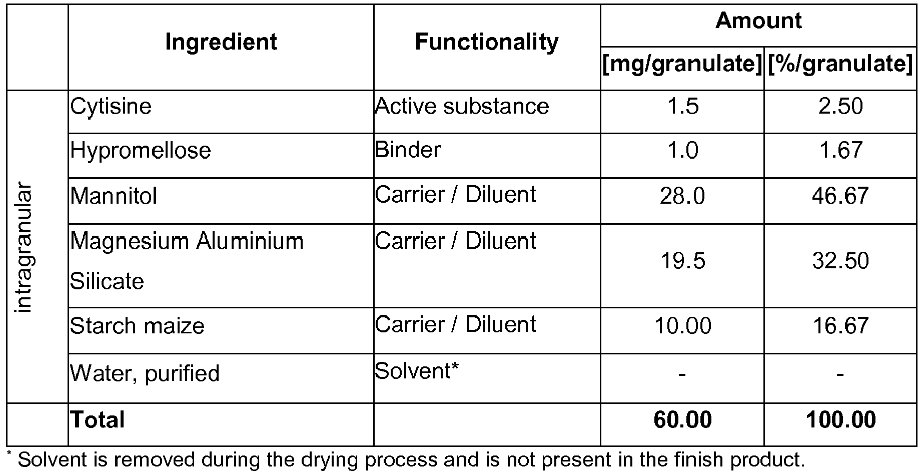

### Example 3D.

Granulate was prepared in a manner described in Example 1 from the following intragranular ingredients.

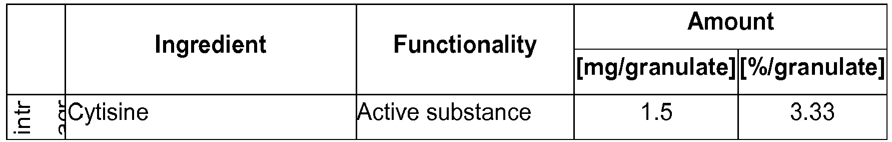

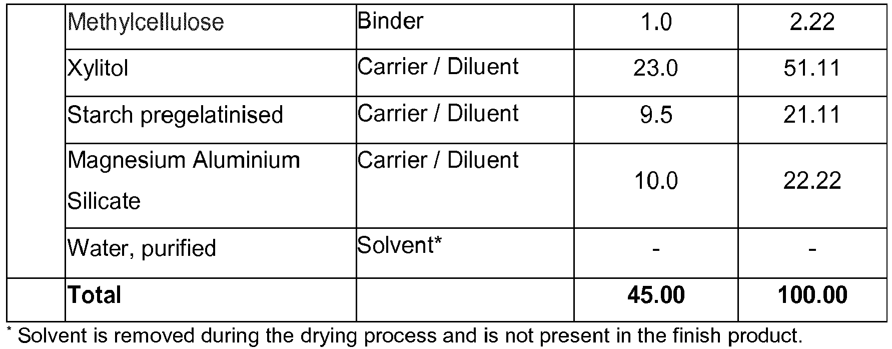

### Example 3E.

Granulate was prepared in a manner described in Example 1 from the following intragranular ingredients.

| | **Ingredient** | **Functionality** | **Amount** | |
|---|---|---|---|---|
| | | | **[mg/granulate]** | **[%/granulate]** |
| Intragranular phase | Cytisine | Active substance | 1.5 | 3.33 |
| | Polyvinyl alcohol | Binder | 0.5 | 1.11 |
| | Calcium hydrogen phosphate anhdyrous | Carrier / Diluent | 13.0 | 28.88 |
| | Starch maize | Carrier / Diluent | 10.0 | 22.22 |
| | Magnesium Aluminium Silicate | Carrier / Diluent | 20.0 | 44.44 |
| | Water, purified | Solvent | - | - |
| | **Total** | | **45.00** | **100.00** |

| | | | | |
|---|---|---|---|---|
| * Solvent is removed during the drying process and is not present in the finish product. | | | | |

### Example 4 (comparative) Preparation of tablets by direct compression

Reference formulation was tested in direct compression technology with two different grades of Cytisine. Required quality was not obtained. Exemplary tested composition is specified in Table 4.

**Table 4. Qualitative and quantitative composition of cytisine tablet core, 1.5 mg**

| **Component** | **Quantity** | |
|---|---|---|
| | **[mg/tab]** | **[%]** |
| Cytisine (grade 1 or 2)* | 1.50 | 2.50 |
| Mannitol | 40.00 | 66.67 |
| Starch pregelatinised | 8.70 | 14.50 |
| Hypromellose | 2.30 | 3.83 |
| Magnesium Aluminium Silicate | 5.70 | 9.50 |
| Sillica colloidal anhydrous | 0.60 | 1.00 |
| Magnesium stearate | 1.20 | 2.00 |
| **Total mass of tablet core** | **60.0** | **100.0** |

| | Grade 1 | Grade 2 |
|---|---|---|
| PSD of Cytisine | d 0.9 = 45 um | D 0.9 = 10 um |

| | | |
|---|---|---|
| *one grade was applied for one product batch | | |

Manufacturing process comprised the following steps:
1) Weighing
2) Blending
3) Tableting by direct compression.

Geometric dilution technique as presented in Table 5 below was used for blending.

**Table 5. Blending procedure**

| **Step** | **Excipients** | **Mixing** | **Result** | **Speed [rpm]** | **Time [min]** |
|---|---|---|---|---|---|
| 1 | Magnesium Aluminium Silicate + Mannitol | manual | Premix I | - | 5 |
| 2 | Cytisine + 10 gram of Premix I | manual | Premix II | - | 5 |
| 3 | Premix II + 20 gram of Premix I + Silica colloidal anhydrous + Hypromellose + Starch pregelatinised | manual | Premix III | - | 5 |
| 4 | Premix III + 50 gram of Premix I | Diffusion Mixer (Tumble) | Premix IV | 12 | 10 |
| 5 | Premix IV + the rest of Premix I | Diffusion Mixer (Tumble) | Blend without lubricant | 12 | 10 |
| 6 | Blend without lubricant + Magnesium stearate | Diffusion Mixer (Tumble) | Final blend (300 gram) | 12 | 5 |

Average content and uniformity of content (as relative standard deviation - RSD) of the tablets were tested. The results are presented in Table 6 below.

**Table 6. Average content and uniformity of content for Cytisine directly compressed tablets.**

| **Cytisine grade** | **Blend** | | **Tableting process stage** | | | |
|---|---|---|---|---|---|---|
| | | | **Beginning** | | **End** | |
| | **Average content [%]** | **RSD [%]** | **Average content [%]** | **RSD [%]** | **Average content [%]** | **RSD [%]** |
| 1 | 96.6 | 5.8 | 88.7 | 2.9 | 103.8 | 8.6 |
| 2 | 100.1 | 5.1 | 95.3 | 2.7 | 104.9 | 5.4 |

As shown above, required quality could not be obtained both for the blend and the tablet even in a small scale due to segregation. The increasing trend of cytisine content for tablets from different stages of tableting process was observed due to segregation. Therefore, the direct compression technology does not provide required quality of the product, especially uniformity of the content, even when geometric dilution technique and quite long mixing time are used, and despite using excipients of improved flowability (magnesium aluminium silicate or silica colloidal anhydrous).

## Claims

1. A solid pharmaceutical composition in the form of a granulate comprising cytisine, a binder and a porous carrier.

2. The solid pharmaceutical composition according to claim 1 wherein the amount of cytisine is in the range from 0.5 to 10% by weight with respect to the weight of the granulate.

3. The solid pharmaceutical composition according to claim 1 or 2 wherein the amount of the porous carrier is in the range from 10 to 92% by weight with respect to the total weight of the granulate.

4. The solid pharmaceutical composition according to any one of claims 1 to 3 wherein the carrier in the granulate of the invention is free of lactose and microcrystalline cellulose.

5. The solid pharmaceutical composition according to any one of claims 1 to 4 wherein the porous carrier is an inorganic one, such as magnesium aluminum silicate, silica, colloidal silica, colloidal silica, or titanium dioxide.

6. The solid pharmaceutical composition according to claim 5 wherein the inorganic porous carrier is magnesium aluminum silicate.

7. The solid pharmaceutical composition according to any one of claims 1 to 4 wherein the porous carrier is an organic, preferably ethylene vinyl acetate copolymer or polypropylene foam powder.

8. The solid pharmaceutical composition according to any one of claims 1 to 7 obtained by wet granulation using a solution of cytisine in a solvent.

9. A solid pharmaceutical composition in the form of a blend of the granulate as defined in any one of claims 1 to 8 with extragranular pharmaceutical excipients.

10. A unit dosage form selected from the group consisting of a tablet, coated tablet, capsule and sachet comprising the solid pharmaceutical composition in the form of a granulate as defined in any one of claim 1 to 8 or in claim 9.

11. A process for the preparation of cytisine solid pharmaceutical composition, said process comprising
a) preparing a granulating liquid by dissolution of cytisine and a binder in a solvent; and
b) granulating a porous carrier with the granulating liquid to prepare the granulate as defined in any one of claims 1 to 7.

12. The process according to claim 11 comprising further step c) wherein extragranular excipients are added to and mixed with the granulate to obtain a blend.

13. The process according to claim 11 or 12 wherein the solvent is water.

14. The process according to claim 11 or 12 wherein the solvent is an organic solvent, preferably ethanol.

15. The process according to any one of claims 11 to 14 wherein in step b) granulating liquid is sprayed onto the carrier in a high-shear mixer.
